# EUROPEAN PATENT APPLICATION

(11) **EP 3 115 440 A1**
(43) Date of publication of application: **11.01.2017**
(21) Application number: 14884315.4
(22) Date of filing: 05.03.2014
(51) Int. Cl.: C10J 3/78, B01J 3/02, B09B 3/00, C02F 11/08

(54) **APPARATUS FOR GASIFICATION WITH SUPERCRITICAL FLUID**

(71) Applicant: The Chugoku Electric Power Co., Inc., Hiroshima-shi, Hiroshima 730-8701 (JP); Hiroshima University, Higashi-Hiroshima-shi Hiroshima 739-8511 (JP); Toyo Koatsu Co., Ltd., Hiroshima 733-0022 (JP)
(72) Inventor: WADA, Yasutaka, Hiroshima-shi Hiroshima 730-8701 (JP); KUBOTA, Haruhito, Hiroshima-shi Hiroshima 730-8701 (JP); YAMAMURA, Yukimasa, Hiroshima-shi Hiroshima 730-8701 (JP); UCHIYAMA, Ichiro, Hiroshima-shi Hiroshima 730-8701 (JP); OYAMA, Keiji, Hiroshima-shi Hiroshima 730-8701 (JP); YAMASAKI, Toshiki, Hiroshima-shi Hiroshima 730-8701 (JP); MATSUMURA, Yukihiko, Higashihiroshima-shi Hiroshima 739-8511 (JP); KAWAI, Yoshifumi, Hiroshima-shi Hiroshima 734-0001 (JP); NOGUCHI, Takashi, Hiroshima-shi Hiroshima 733-0002 (JP)
(74) Representative: Osha Liang
(86) International application number: PCT/JP2014/055695
(87) International publication number: WO 2015/132922

(57) **Abstract**

[PROBLEM]

In a gasification apparatus that heats and pressurizes a gasification feedstock to bring the gasification feedstock into a fluid in a supercritical state and performs decomposition-treatment on the gasification feedstock to obtain fuel gas, energy possessed by the treated fluid can be effectively utilized.

[SOLUTION]

A gasification apparatus configured to heat and pressurize a gasification feedstock to bring the gasification feedstock into a supercritical state, and perform decomposition-treatment on the gasification feedstock to obtain fuel gas, the gasification apparatus including: a heat exchanger 31 configured to introduce the gasification feedstock into a low-temperature-side flow channel 31a and introduce treated fluid in a supercritical state into a high-temperature-side flow channel 31b, so that heat exchange is performed between the gasification feedstock and the treated fluid; a gas-liquid separator 51 configured to extract, from the high-temperature-side flow channel 31b, the treated fluid that has been in a subcritical state due to heat exchange, perform gas-liquid separation on the treated fluid, and return a separated liquid to the high-temperature-side flow channel 31b; and a synthesis device 52 configured to synthesize a liquid fuel from fuel gas separated by the gas-liquid separator 51.

## Description

### [Technical Field]

The present invention relates to a gasification apparatus that heats and pressurizes a gasification feedstock to bring the gasification feedstock into a fluid in a supercritical state and performs decomposition-treatment on the gasification feedstock to obtain fuel gas.

### [Background Art]

Gasification apparatuses are known that perform decomposition-treatment on a gasification feedstock in a supercritical state to obtain fuel gas. For example, Patent Literature 1 describes a biomass gasification power generation system in which biomass slurry containing a non-metal catalyst is subjected to hydrothermal treatment under conditions of a temperature of 374°C or greater and a pressure of 22.1 MPa or greater, power is generated by a power generating device using the produced gas that is produced, and waste heat from the power generating device is used to heat the slurry.

Patent Literature 2 describes a technique in which biomass is gasified and compressed, the compressed biomass gas is supplied to a liquid fuel reaction vessel loaded with a catalyst, and liquid fuel is synthesized using waste heat.

### [Citation List]

### [Patent Literature]

Patent Literature 1: Japanese Patent Application Laid-open Publication No. 2008-246343
Patent Literature 2: Japanese Patent Application Laid-open Publication No. 2010-174153

### [Summary of Invention]

### [Technical Problem]

In the system of Patent Literature 1, a treated fluid that has been subjected to gasification treatment exchanges heat with the slurry in a double-pipe heat exchanger. The treated fluid thereby transitions from a supercritical state to a subcritical state, and changes from a mixed gas-liquid state to a gas-liquid two-phase flow.

Since the gas-liquid two-phase flow vertically separates, with the gas (such as fuel gas) and the liquid having a volume ratio of approximately 2:8, the energy possessed by the treated fluid was not being effectively utilized. For example, in spite of the fact that the gas has pressure energy and can also be used as a fuel, the heat exchange efficiency has been lowered due to using the gas in heat exchange.

The present invention has been made in view of the above issue, and an object of the present invention is to effectively utilize energy possessed by the treated fluid.

### [Solution to Problem]

In order to achieve the above object, the present invention is a gasification apparatus configured to heat and pressurize a gasification feedstock to bring the gasification feedstock into a supercritical state, and perform decomposition-treatment on the gasification feedstock to obtain fuel gas, the gasification apparatus including: a heat exchanger configured to introduce the gasification feedstock into a low-temperature-side flow channel and introduce treated fluid in a supercritical state into a high-temperature-side flow channel, so that heat exchange is performed between the gasification feedstock and the treated fluid; a gas-liquid separator configured to extract, from the high-temperature-side flow channel, the treated fluid that has been in a subcritical state due to heat exchange, perform gas-liquid separation on the treated fluid, and return a separated liquid to the high-temperature-side flow channel; and a synthesis device configured to synthesize a liquid fuel from fuel gas separated by the gas-liquid separator.

According to the present invention, treated fluid in a subcritical state is extracted from the high-temperature-side flow channel, and is gas-liquid separated. Liquid fuel is produced from the fuel gas that has been gas-liquid separated, enabling the energy possessed by the fuel gas to be utilized effectively. Moreover, liquid that has been gas-liquid separated is returned to the high-temperature-side flow channel, enabling the heat exchange efficiency with the gasification feedstock to be enhanced by the returned liquid.

In the above-described gasification apparatus, the synthesis device is preferably configured to cause carbon monoxide and hydrogen contained in the fuel gas to catalytically react with each other under a high pressure to produce methanol. In such a configuration, since the fuel gas that has been gas-liquid separated is in a high pressure state, compression of the fuel gas can be omitted when methanol is being produced. This thereby enables the efficiency of production of methanol to be improved.

In the above-described gasification apparatus, the fuel gas that has not been used in production of the liquid fuel is preferably used to heat the gasification feedstock that has exchanged heat in the heat exchanger. In such a configuration, due to the gasification feedstock being heated by the remaining fuel gas, energy possessed by the fuel gas can be even more effectively utilized.

### [Advantageous Effects of Invention]

According to the present invention, energy possessed by the treated fluid can be effectively utilized in a gasification apparatus that heats and pressurizes a gasification feedstock to make it into a fluid in a supercritical state and performs decomposition-treatment on the gasification feedstock to obtain fuel gas.

### [Brief Description of Drawings]

[Fig. 1] Fig. 1 is a diagram illustrating a configuration of a supercritical gasification apparatus.
[Fig. 2] Fig. 2 is a diagram illustrating a configuration of a gas-liquid separator.
[Fig. 3A] Fig. 3A is a diagram illustrating a state in a double-pipe heat exchanger before gas-liquid separation.
[Fig. 3B] Fig. 3B is a diagram illustrating a state in a double-pipe heat exchanger after gas-liquid separation.

### [Description of Embodiments]

Embodiments of the present invention will be described below.

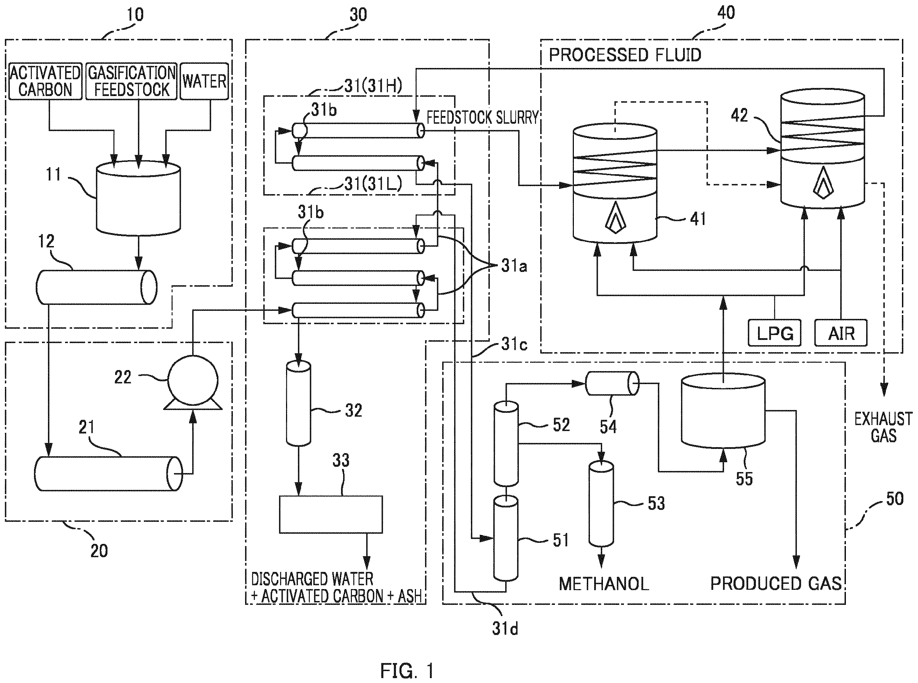

First, explanation follows regarding an overall configuration of a supercritical gasification apparatus, with reference to Fig. 1. The exemplified supercritical gasification apparatus includes a feedstock regulation unit 10, a feedstock supply unit 20, a heat exchange unit 30, a gasification treatment unit 40, and a liquid fuel production unit 50.

In the supercritical gasification apparatus, the feedstock supply unit 20 feeds out, at high pressure, a feedstock slurry regulated by the feedstock regulation unit 10 to a low-temperature-side flow channel 31a of a heat exchanger 31 included in the heat exchange unit 30. The feedstock slurry heated by the heat exchange unit 30 is then further heated by the gasification treatment unit 40 and is brought into a supercritical state. Then, organic matter contained in the feedstock slurry is subjected to decomposition-treatment to produce fuel gas that contains hydrogen, methane, ethane, ethylene, carbon monoxide, and the like.

Treated fluid in a supercritical state is introduced to a high-temperature-side flow channel 31b of the heat exchanger 31, and exchanges heat with the feedstock slurry. This heat exchange brings the treated fluid into a subcritical state, and changes the treated fluid into a gas-liquid two-phase flow. Then, in the middle of the high-temperature-side flow channel 31b, the treated fluid in the subcritical state is extracted from the heat exchanger 31, and is gas-liquid separated by the liquid fuel production unit 50. The liquid that has been gas-liquid separated is then returned to the high-temperature-side flow channel 31b of the heat exchanger 31, and is used in heat exchange with the feedstock slurry. On the other hand, the gas (fuel gas) that has been gas-liquid separated produces liquid fuel which is then also employed as a fuel for the gasification treatment unit 40.

Explanation follows regarding each section of the supercritical gasification apparatus.

The feedstock regulation unit 10 is a section that regulates feedstock slurry from a gasification feedstock or the like, and includes a regulation tank 11 and a crusher 12.

The regulation tank 11 is a container that mixes the gasification feedstock, activated carbon, water and the like to produce a suspension, and is provided with stirring blades, not shown in the drawings. As the gasification feedstock, Shochu residue, egg-laying hen droppings, or sludge, for example, may be employed. The activated carbon functions as a non-metal catalyst, and porous particles of activated carbon having an average particle diameter of 200 µm or less may be employed therefor.

The crusher 12 is a device that crushes solid components (primarily the gasification feedstock) contained in the suspension mixed in the regulation tank 11, so as to make the solid components into a uniform size. In the present embodiment, crushing is performed such that the average particle diameter of the solid components becomes 500 µm or less. The suspension becomes a feedstock slurry by crushing with the crusher 12.

The feedstock supply unit 20 is a section that feeds the feedstock slurry out at high pressure, and includes a supply pump 21 and a high pressure pump 22. The supply pump 21 is a device for supplying the feedstock slurry fed out from the crusher 12 toward the high pressure pump 22. The high pressure pump 22 is a device for feeding the feedstock slurry out at high pressure. The feedstock slurry is pressurized by the high pressure pump 22 to a pressure of from approximately 0.1 MPa to approximately 4 MPa, inclusive.

The heat exchange unit 30 is a section that causes heat exchange to be performed between the feedstock slurry supplied from the feedstock supply unit 20 and the treated fluid that has been decomposition-treated by the gasification treatment unit 40, such that the feedstock slurry is heated while the treated fluid is cooled. The heat exchange unit 30 includes a heat exchanger 31, a depressurizing mechanism 32, and a cooler 33.

The heat exchanger 31 is a device that causes heat exchange to be performed between the feedstock slurry and the treated fluid, and a double-pipe structure is employed therefor. An inner flow channel is employed as the low-temperature-side flow channel 31a through which the feedstock slurry flows, and an outer flow channel is employed as the high-temperature-side flow channel 31b through which the treated fluid flows. In the present embodiment, the treated fluid is introduced at a temperature of approximately 600°C and is discharged at a temperature of approximately 120°C. On the other hand, the feedstock slurry is introduced at a temperature of room temperature and discharged at a temperature of approximately 450°C. Note that description regarding the heat exchanger 31 is given later.

The depressurizing mechanism 32 is a device that depressurizes the treated fluid discharged from the heat exchanger 31. The cooler 33 is a device that cools the treated fluid discharged from the depressurizing mechanism 32. By the depressurizing mechanism 32 and the cooler 33, the treated fluid discharged from the cooler 33 (a mixture of discharged water, activated carbon, and ash) is depressurized and cooled to approximately room temperature and pressure.

The gasification treatment unit 40 is a section that heats and pressurizes the feedstock slurry heated by the heat exchanger 31 until the feedstock slurry reaches a supercritical state, and decomposes organic matter contained in the feedstock slurry. The gasification treatment unit 40 includes a preheater 41 and a gasification reactor 42. The preheater 41 is a device that preheats the feedstock slurry discharged from the heat exchanger 31. In the present embodiment, feedstock slurry introduced at approximately 450°C is heated to approximately 600°C. The gasification reactor 42 is a device that maintains the feedstock slurry in a supercritical state so as to decompose organic matter contained in the feedstock slurry. In the present embodiment, decomposition-treatment is performed on the feedstock slurry for a duration of from 1 minute to 2 minutes, with the temperature set to 600°C and the pressure set to 25 MPa.

The liquid fuel production unit 50 recovers fuel gas from the treated fluid, and produces liquid fuel (methanol in the present embodiment), while residual fuel gas is also employed as a fuel in the gasification treatment unit 40. The liquid fuel production unit 50 includes a gas-liquid separator 51, a synthesis device 52, a temperature lowering device 53, a depressurizing mechanism 54, and a gas tank 55.

The gas-liquid separator 51 is a section that separates the treated fluid in a subcritical state, extracted from the middle of the high-temperature-side flow channel 31b (the outer flow channel) provided to the heat exchanger 31, into a gas (fuel gas) and a liquid (discharged water, activated carbon, and ash). The separated liquid is then returned to the high-temperature-side flow channel 31b of the heat exchanger 31, and the separated gas is supplied to the synthesis device 52. Note that explanation regarding the gas-liquid separator 51 is given later.

The synthesis device 52 is a section that synthesizes liquid fuel from the separated gas (fuel gas), and uses carbon monoxide and hydrogen contained in the gas to synthesize methanol, which is a type of liquid fuel. In the present embodiment, the fuel gas is discharged from the gas-liquid separator 51 at a temperature of approximately 300°C and a pressure of approximately 25 MPa, so that at this temperature and pressure a catalytic reaction is performed in the synthesis device 52 in the presence of a zinc oxide-chromic acid catalyst. The temperature lowering device 53 is a section that lowers the temperature of reactants discharged from the synthesis device 52 and liquefies the reactants discharged from the synthesis device 52. Liquefied reactants lowered in temperature by the temperature lowering device 53 are recovered as methanol (liquid fuel).

The depressurizing mechanism 54 is a device that depressurizes the gas discharged from the synthesis device 52. Namely, the depressurizing mechanism 54 is a device that reduces the pressure of fuel gas that has been not used in production of liquid fuel. The gas tank 55 is a container that accumulates fuel gas that has been depressurized by the depressurizing mechanism 54. The fuel gas accumulated in the gas tank 55 is then supplied as a part of the fuel for the preheater 41 and the gasification reactor 42 included in the gasification treatment unit 40.

Next, explanation follows regarding extraction of fuel gas from the treated fluid using the heat exchanger 31 and the gas-liquid separator 51.

The heat exchanger 31 is configured so as to be separated into a high-temperature-side section 31H and a low-temperature-side section 31L. Treated fluid in a high temperature and high pressure state (600°C, 25 MPa in the present embodiment) is introduced to the high-temperature-side section 31H, and exchanges heat with the feedstock slurry discharged from the low-temperature-side section 31L. On the other hand, room temperature feedstock slurry pressurized by the high pressure pump 22 is introduced to the low-temperature-side section 31L, and exchanges heat with the treated fluid (the liquid component) that has been gas-liquid separated.

The treated fluid that has exchanged heat in the high-temperature-side section 31H is lowered in temperature while being maintained at high pressure, and transitions to a subcritical state. For example, the temperature is lowered to approximately 300 °C while maintaining the pressure at 25 MPa. When the temperature is lowered, the treated fluid is brought into a subcritical state and changes into a gas-liquid two-phase flow. As described above, the treated fluid in a subcritical state is then extracted from the heat exchanger 31, and is gas-liquid separated by the gas-liquid separator 51.

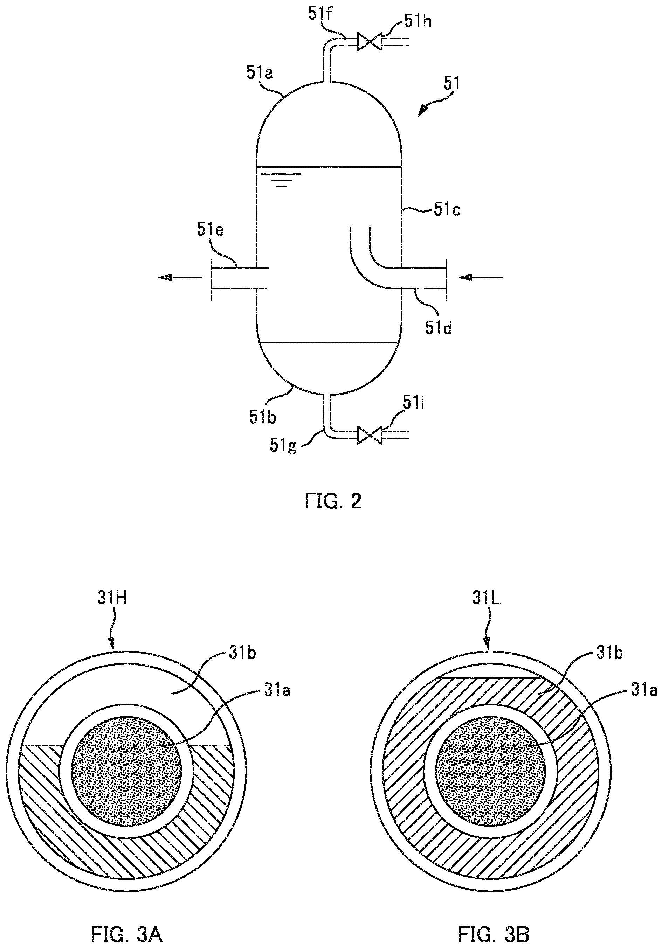

Fig. 2 is a vertical cross-section of the gas-liquid separator 51. The gas-liquid separator 51 given as an example is a sealed container having an upper end portion 51a and a lower end portion 51b that are both semi-spherical in shape, and an intermediate portion 51c that is a cylindrical shape. A fluid introduction portion 51d and a liquid discharge portion 51e are provided on a side face of the intermediate portion 51c. A gas discharge portion 51f is provided to the upper end portion 51a, and a drain 51g is provided to the lower end portion 51b.

The fluid introduction portion 51d is a pipe shaped member that communicates with the interior and exterior of the gas-liquid separator 51. An outside end portion of the fluid introduction portion 51d is connected to the high-temperature-side flow channel 31b provided to the high-temperature-side section 31H, through piping 31c (see Fig. 1). The liquid discharge portion 51e is also a pipe shaped member that communicates with the interior and exterior of the gas-liquid separator 51. An outside end portion of the liquid discharge portion 51e is connected to the high-temperature-side flow channel 31b provided to the low-temperature-side section 31L, through piping 31d (see Fig. 1).

The gas discharge portion 51f is configured by piping having a base end that is communicated with a space inside the gas-liquid separator 51, and a leading end thereof is provided with an opening and closing valve 51h. The gas discharge portion 51f is communicated with a flow rate adjusting mechanism through piping. The drain 51g is also configured with piping having a base end that is communicated with the space inside the gas-liquid separator 51, and a leading end portion thereof is provided with a drain valve 51i.

In the gas-liquid separator 51, treated fluid (a gas-liquid two-phase flow) discharged from the high-temperature-side section 31H flows into the space inside the gas-liquid separator 51. In the interior space, the treated fluid is separated into a liquid component (activated carbon, water, ash, and tar) and a gas component (fuel gas). The gas component then rises and flows into the gas discharge portion 51f from the upper end portion 51a. The fuel gas is subsequently supplied to the synthesis device 52. Note that, pressure regulation is not performed in the gas-liquid separator 51. Fuel gas at a high pressure of approximately 25 MPa is thereby supplied to the synthesis device 52.

The synthesis device 52 can accordingly use the fuel gas in methanol synthesis without compressing the fuel gas. Moreover, as described above, the fuel gas can be used in methanol synthesis without being heated since the fuel gas has a temperature of around 300°C. It is known that a great amount of energy is required when compressing or heating a gas from the outside. In relation to this point, methanol can be synthesized using little energy in the supercritical gasification apparatus of the present embodiment since the fuel gas discharged from the gas-liquid separator 51 is at a temperature and pressure suitable for methanol synthesis.

On the other hand, the separated liquid component fills up a space inside the gas-liquid separator 51 from the lower side thereof, and is discharged from the liquid discharge portion 51e. Note that, although tar is also contained in the liquid component, the tar precipitates due to having a higher specific gravity than water, activated carbon, or ash, and the tar is collected in the lower end portion 51b of the interior space. Tar accumulated in the gas-liquid separator 51 can thereby be recovered by opening the drain valve 51i.

A liquid component from which the fuel gas and tar have been removed is thus discharged from the liquid discharge portion 51e. For the sake of convenience in the following description, the liquid component from which the fuel gas and the tar have been removed is referred to as the treated fluid discharged from the gas-liquid separator 51. The treated fluid is employed to heat the feedstock slurry in the low-temperature-side section 31L of the heat exchanger 31.

As illustrated in Fig. 3A, in the high-temperature-side section 31H of the heat exchanger 31, gas of the treated fluid in a subcritical state has tended to be collected in an upper portion of the outer flow channel, and this has impaired the heat exchange efficiency with the feedstock slurry. However, gas has been removed from the treated fluid discharged from the gas-liquid separator 51. Therefore, as illustrated in Fig. 3B, the treated fluid fills the entire high-temperature-side flow channel 31b, and highly efficient heat exchange with the feedstock slurry flowing through the low-temperature-side flow channel 31a is achieved. This enables the feedstock slurry to be efficiently heated in the low-temperature-side section 31L of the heat exchanger 31.

As is apparent from the above description, in the supercritical gasification apparatus of the present embodiment, treated fluid in a subcritical state is extracted from the high-temperature-side flow channel 31b provided to the high-temperature-side section 31H, and is gas-liquid separated. Since liquid fuel (methanol) is then produced from the high pressure fuel gas that has been gas-liquid separated, the fuel gas can be effectively utilized as new energy. Moreover, fuel gas that has not employed in liquid fuel production can be effectively utilized as a fuel for the gasification treatment unit 40.

Moreover, the treated fluid that has been gas-liquid separated is returned to the high-temperature-side flow channel 31b provided to the low-temperature-side section 31L, enabling more efficient heat exchange between the returned treated fluid and the feedstock slurry. Moreover, blockages in the heat exchanger 31 caused by tar can be suppressed due to being able to remove the tar in the gas-liquid separation process.

The embodiment described above is to facilitate understanding of the present invention, and does not limit the present invention. The present invention may be modified and improved without departing from the gist thereof, and the present invention includes equivalents thereof. For example, although an example was given in the embodiment described above in which methanol serves as the liquid fuel that is produced in the liquid fuel production unit 50, configuration may be made such that another type of liquid fuel is produced.

### [Reference Signs List]

10: feedstock regulation unit, 11: regulation tank, 12: crusher, 20: feedstock supply unit, 21: supply pump, 22: high pressure pump, 30: heat exchange unit, 31: heat exchanger, 31H: high-temperature-side section, 31L: low-temperature-side section, 31a: low-temperature-side flow channel, 31b: high-temperature-side flow channel, 32: depressurizing mechanism, 33: cooler, 40: gasification treatment unit, 41: preheater, 42: gasification reactor, 50: liquid fuel production unit, 51: gas-liquid separator, 51a: gas-liquid separator upper end portion, 51b: gas-liquid separator lower end portion, 51c: gas-liquid separator intermediate portion, 51d: fluid introduction portion, 51e: liquid discharge portion, 51f: gas discharge portion, 51g: drain, 51h: opening and closing valve of gas discharge portion, 51i: drain valve, 52: synthesis device, 53: temperature lowering device, 54: depressurizing mechanism, 55: gas tank

## Claims

1. A gasification apparatus configured to heat and pressurize a gasification feedstock to bring the gasification feedstock into a supercritical state, and perform decomposition-treatment on the gasification feedstock to obtain fuel gas, the gasification apparatus comprising:
a heat exchanger configured to introduce the gasification feedstock into a low-temperature-side flow channel and introduce treated fluid in a supercritical state into a high-temperature-side flow channel, so that heat exchange is performed between the gasification feedstock and the treated fluid;
a gas-liquid separator configured to extract, from the high-temperature-side flow channel, the treated fluid that has been in a subcritical state due to heat exchange, perform gas-liquid separation on the treated fluid, and return a separated liquid to the high-temperature-side flow channel; and
a synthesis device configured to synthesize a liquid fuel from fuel gas separated by the gas-liquid separator.

2. The gasification apparatus according to claim 1, wherein
the synthesis device is configured to cause carbon monoxide and hydrogen contained in the fuel gas to catalytically react with each other under a high pressure to produce methanol.

3. The gasification apparatus according to claim 1 or claim 2, wherein
the fuel gas that has not been used in production of the liquid fuel is used to heat the gasification feedstock that has exchanged heat in the heat exchanger.
